# EUROPÄISCHE PATENTANMELDUNG

(11) **EP 0 568 836 A1**
(43) Veröffentlichungstag der Anmeldung: **10.11.1993**
(21) Anmeldenummer: 93105854.9
(22) Anmeldetag: 08.04.1993
(51) Int. Cl.: C07K 15/00, C07K 15/06

(54) **Reinigung von Plasminogen-Aktivator-Inhibitor 2 durch Immunaffinitätschromatographie**

(30) Priorität: 06.05.1992 DE 4214999
(71) Anmelder: BEHRINGWERKE Aktiengesellschaft, 35001 Marburg (DE)
(72) Erfinder: Hock, Johann, W-3550 Marburg (DE)

(57) **Zusammenfassung**

Die Erfindung betrifft ein Verfahren zur immunaffinitätschromatographischen Reinigung von Plasminogen-Aktivator-Inhibitor 2 (PAI-2) und monoklonale Antikörper, die für ein solches Verfahren geeignet sind.

## Beschreibung

Die Erfindung betrifft ein verfahren zur immunaffinitätschromatographischen Reinigung von Plasminogen-Aktivator-Inhibitor 2 (PAI-2) und monoklonale Antikörper, die für ein solches Verfahren geeignet sind.

Plasmin ist eine zentrale Protease für viele physiologische Prozesse wie z. B. Fibrinolyse, Zellmigration, Zelldifferenzierung oder Gewebsumbau. Die Regulation der Plasminaktivität erfolgt unter anderem über die proteolytische Umwandlung des Proenzyms Plasminogen in die aktive Protease Plasmin durch die Plasminogenaktivatoren tPA (tissue type plasminogen activator) und Urokinase. Die Aktivität dieser Proteasen unterliegt wiederum der Kontrolle spezifischer Inhibitoren wie PAI-1 oder PAI-2.

PAI-2 wird unter anderem in menschlichem Placentagewebe, im Plasma von Schwangeren und in Monocyten gefunden. Aus Placenta wird PAI-2 als nicht glykosyliertes Protein isoliert und erscheint in der SDS-Polyacrylamid-Gelelektrophorese als Doppelbande mit Molekulargewichten von 45.000 bzw. 51.000 Dalton. Durch Reduktion von Disulfidbrucken kann die 51.000 Dalton-Form in die 45.000 Dalton-Form überführt werden. Die 51.000 Dalton-Form stellt somit ein Oxidationsprodukt der 45.000 Dalton-Form dar. Ein Teil des PAI-2 liegt in der Placenta in Form von Komplexen mit anderen Proteinen, z.B. Vitronectin, vor. In gereinigten Präparationen werden in der SDS-Polyacrylamid-Gelelektrophorese auch Oligomere von PAI-2 gefunden, die unter reduzierenden Bedingungen in die monomere Form überführt werden können. Aus Schwangerenplasma wird PAI-2 als glykosyliertes Protein mit einem Molekulargewicht von ca. 65.000 Dalton isoliert, während aus Monocytenkulturen sowohl die glykosylierte als auch die nicht glykosylierte Form gewonnen werden kann. Alle beschriebenen Formen von PAI-2 sind in der Lage, Urokinase zu inhibieren. Unter PAI-2 werden im folgenden alle inhibitorisch aktiven Formen (nicht glykosyliert, glykosyliert, Oligomere und Komplexe mit anderen Proteinen) verstanden.

Bei der Reinigung von Proteinen aus humanen Geweben oder Körperflüssigkeiten für therapeutische Zwecke bringt eine hohe Reinheit ein geringeres Risiko der Virusübertragung mit sich. Die Reinigung rekombinanter humaner Proteine aus Kulturen von Mikroorganismen für therapeutische Zwecke muß zu einer sehr hohen Reinheit dieser Proteine führen, um sicherzustellen, daß antigen wirkende Proteine der Wirtszellen möglichst vollständig entfernt werden. Die Affinitätschromatographie mit monoklonalen Antikörpern ist eine Methode, die das Erreichen einer extrem hohen Reinheit ermöglicht. Da die Reinigung auch aus komplexen Proteingemischen mit Hilfe der Immunaffinitätschromatographie nur wenige Reinigungsschritte erfordert, ist auch eine höhere Ausbeute als bei herkömmlichen Verfahren zu erzielen.

Die Reinigung von PAI-2 mittels Immunaffinitätschromatographie wurde bereits beschrieben (Åstedt,B. et al. (1985) Thromb. Haemost. 53:122-125). Die verwendeten Elutionsbedingungen, nämlich 2 mol/1 KSCN, 0.01% Triton, bewirken jedoch eine zumindest partielle Denaturierung von PAI-2. Das beschriebene Verfahren ist somit nicht geeignet, um nativen PAI-2 z.B. für therapeutische Zwecke zu gewinnen.

Aufgabe der Erfindung ist daher die Entwicklung eines Verfahrens für die immunaffinitätschromatographische Reinigung von nativem PAI-2 aus Ausgangsmaterialien wie Placenta, Monocyten oder Extrakten oder Kulturüberständen von rekombinanten Zellen, welche die genetische Information für PAI-2 enthalten.

Überraschenderweise wurde gefunden, daß es möglich ist, PAI-2 an immobilisierte monoklonale Antikörper zu binden und durch wässrige Lösungen mit geringer Ionenstärke oder mit destilliertem Wasser in aktiver Form zu eluieren.

Gegenstand der Erfindung ist ein Verfahren zur Reinigung von nativem PAI-2 mittels Immunaffinitäts-Chromatographie, dadurch gekennzeichnet, daß eine Lösung, die PAI-2 enthält, mit einem an ein unlösliches Träger-Material gebundenen monoklonalen Antikörper gegen PAI-2 in Kontakt gebracht, Affinitätsmaterial und Flüssigkeit von einander getrennt und PAI-2 vom Affinitätsmaterial in biologisch aktiver Form eluiert wird.

Gegenstand der Erfindung sind auch monoklonale Antikörper gegen PAI-2, die für die Reinigung von nativem PAI-2 mittels Immunaffinitätschromatographie geeignet sind. Solche Antikörper sind gekennzeichnet dadurch, daß PAI-2 spezifisch an sie gebunden wird und unter schonenden, d. h. die biologische Aktivität nicht beeinträchtigenden Bedingungen wieder dissoziiert werden kann. Monoklonale Antikörper im Sinne der Erfindung sind auch die dem Fachmann an sich bekannten immunreaktiven Fragmente monoklonaler Antikörper wie z.B. F(ab')2- , Fab- oder Fv-Fragmente und antigen-bindende Einzelketten von Antikörper-Molekülen.

Geeignete monoklonale Antikörper können nach Köhler und Milstein (Nature 256:285-308) oder einer der vielen Varianten ihrer Methode (z.B. Goding, J.W. (1980), J.Immunol.Meth. 39:285-308), die dem Fachmann an sich bekannt sind, hergestellt werden, beispielsweise auf folgende Weise:

Säugetiere, bevorzugt Mäuse oder Ratten, werden durch mehrere Injektionen im Abstand von jeweils 1-4 Wochen mit einer PAI-2 enthaltenden Flüssigkeit, bevorzugt einer Emulsion von gereinigtem PAI-2 in Freund's Adjuvans, immunisiert. Zur Vorbereitung für eine Zellfusion wird PAI-2 3-5 Tage vor dem geplanten Fusionstermin in wässriger Lösung appliziert, bevorzugt intraperitoneal oder intravenös.

Zur Gewinnung antikörper-produzierender Zellen wird ein immunisiertes Tier getötet, ein lymphatisches Organ, vorzugsweise die Milz, entnommen und die Lymphocyten freigesetzt. Um in Zellkultur permanent wachsende antikörper-produzierende Zellen zu erhalten, müssen die Lymphocyten immortalisiert werden. Dies kann auf verschiedene Weisen erfolgen, z.B. durch Transformation mit Epstein-Barr-Virus oder Retroviren. Bevorzugt werden jedoch die Lymphocyten mit Myelomzellen fusioniert. Besonders geeignet sind Myelomzellinien aus BALB/c-Mäusen, die keine Immunglobuline sezernieren, beispielsweise die Zellinien SP2/0-Ag14 oder X63-Ag8.653. Die Zellen können durch Inkubation mit Polyethylenglykol mit einem Molekulargewicht von 1000-6000 in 30-60%iger Lösung fusioniert werden, aber auch andere Verfahren, z.B. Elektrofusion, sind geeignet. Durch Kultivierung in einem geeigneten Nährmedium werden Hybride aus Lymphocyten und Myelomzellen (Hybridome) selektiert und vermehrt. Die Hybridome werden 1-3 Wochen nach der Zellfusion auf Produktion spezifischer Antikörper getestet. Hierfür steht eine Vielzahl von Testsystemen zur Verfügung, die dem Fachmann bekannt sind. Bevorzugt wird ein ELISA-Testsystem verwendet, bei dem PAI-2 an eine Festphase adsorbiert ist. Die Zellüberstände der Hybridome werden zunächst mit PAI-2 in Kontakt gebracht und PAI-2 spezifische Antikörper durch Inkubation mit einem enzymmarkierten Antikörper gegen Maus-IgG und nachfolgenden Zusatz eines chromogenen Substrats für das Markierungsenzym detektiert. Zellen, die spezifische Antikörper gegen PAI-2 produzieren, werden durch Vereinzeln unter mikroskopischer Kontrolle oder durch das Verfahren der limitierenden Verdünnung kloniert. Klonale Zellinien werden für die Antikörpergewinnnung in vitro vermehrt. Monoklonale Antikörper können aus dem verbrauchten Kulturmedium der Zellen gewonnen werden. Hierzu steht eine Vielzahl von Verfahren zur Verfügung, vorzugsweise wird eine Affinitätschromatographie an immobilisiertem Protein A oder PAI-2 durchgeführt.

Die gereinigten monoklonalen Antikörper werden dann auf ihre Eignung für die Immunaffinitätschromatographie getestet. Hierzu werden Antikörper an ein geeignetes unlösliches Trägermaterial nach dem Fachmann vertrauten Verfahren gekoppelt. Als Träger finden in der Affinitätschromatographie verschiedene Materialien Verwendung, z.B. Derivate von Agarose, Polyacrylamid oder Cellulose. Die Antikörper können nach Aktivierung des Trägers z.B. mit Bromcyan oder Carbodiimid gekoppelt werden. Verschiedene weitere Trägermaterialien und Kopplungsmethoden sind dem Fachmann bekannt. Grundsätzlich ist als Träger jedes gebräuchliche Material und jede Kopplungsmethode geeignet, bei dem die Aktivität des gekoppelten Antikörpers nicht oder nur geringfügig beeinträchtigt wird. Die Affinitätsgele werden dann auf ihre Eignung für die Reinigung von PAI-2 getestet. Hierzu wird eine PAI-2 enthaltende Lösung über das Affinitätsgel gepumpt und die PAI-2-Aktivität im Durchlauf bestimmt. Gele, an die PAI-2 adsorbiert wird, sind für die Immunaffinitätschromatographie geeignet.

In einer bevorzugten Verfahrensweise erfolgt die Bindung von PAI-2 an das Immunaffinitätsgel aus einer Lösung mit einer Leitfähigkeit von ≧5 mS bei einem pH-Wert zwischen 5.5 und 8.5 und bei einer Temperatur von +4°C bis +37°C. Besonders bevorzugt enthält der Puffer 0.05 mol/l Phosphat oder Tris und 0-3.0 mol/l NaCl.

Der kritische Schritt ist die Elution von PAI-2 vom Affinitätsgel. Die Dissoziation des Antigen-Antikörper-Komplexes erfordert häufig Bedingungen, unter denen die Aktivität von PAI-2 irreversibel zerstört wird. Es müssen daher schonende Elutionsbedingungen gefunden werden, die eine Dissoziation des Antigen-Antikörper-Komplexes unter Erhaltung der PAI-2-Aktivität erlauben. Hierzu können grundsätzlich verschiedene Elutionsmittel verwendet werden, z.B. organische Lösungsmittel, Detergentien, Lösungen von sehr niedriger oder sehr hoher Ionenstärke oder Kombinationen verschiedener Elutionsmittel. Solche und weitere Elutionsverfahren, (z. B. temperaturabhängige) sind dem Fachmann bekannt. Bevorzugt werden für die Elution von PAI-2 Lösungen mit sehr niedriger Ionenstärke (Leitfähigkeit ≦3 mS) bei einem pH zwischen 5.5 und 8.5, besonders bevorzugt destilliertes Wasser verwendet. Dem Elutionsmittel können verschiedene Substanzen, z. B. Zucker, Zuckeralkohole, Polysaccharide, Aminosäuren oder Proteine zugesetzt werden, um den eluierten PAI-2 zu stabilisieren.

Die folgenden Beispiele erläutern die Erfindung:

### Beispiel 1

### Herstellung und Selektion monoklonaler Antikörper

a) Immunisierung von Mäusen
   Weibliche BALB/c-Mäuse wurden durch subkutane Injektion einer Emulsion von 50 µg PAI-2 (hergestellt wie von Radtke et al. (1990) Biol.Chem.Hoppe-Seyler, 371:1119-1127 beschrieben) in komplettem Freund'schem Adjuvans immunisiert (Tag 1). An den Tagen 28 und 56 wurden je 30 µg PAI-2, emulgiert in inkomplettem Freund'schem Adjuvans, ebenfalls subkutan injiziert. Am Tag 92 folgte eine intraperitoneale Injektion von 100 µg PAI-2 in 0.5 ml physiologischer Kochsalzlösung.
b) Fusion von Lymphocyten mit Myelomzellen
   Am Tag 95 wurden nach Entnahme der Milz durch mechanische Desintegration Lymphocyten gewonnen (ca. 1x10⁸ Zellen). Die Lymphocyten wurden in Dulbecco's Modified Eagle's Medium (DMEM) gewaschen, mit 5x10⁷ Zellen der Myelomzellinie SP2/0-Ag14 gemischt und abzentrifugiert. Nach vollständiger Entfernung des Überstands wurden dem Zellpellet 0.5 ml einer 50%igen Lösung von Polyethylenglykol 4000 in DMEM innerhalb einer Minute zugetropft. Die Suspension wurde 90 Sekunden bei 37°C inkubiert und anschließend durch Zugabe von 7.5 ml DMEM über einen Zeitraum von 5 Minuten verdünnt. Nach einer Inkubation von 10 Minuten bei Raumtemperatur wurde mit DMEM auf 40 ml aufgefüllt, und die Zellen wurden abzentrifugiert. Nach Absaugen des Überstands wurden die Zellen in DMEM mit 20% fetalem Kälberserum (FKS) und 13.6 mg/ml Hypoxanthin, 0.18 mg/ml Aminopterin, 3.9 mg/ml Thymidin (HAT-Medium) resuspendiert und auf 6 Mikrotiterplatten ausgesät (200 µl pro Kavität). Verbrauchtes Medium wurde in Abständen von 3-4 Tagen durch frisches ersetzt, nach 10 Tagen wurde HAT-Medium durch HT-Medium ersetzt.
c) Test auf Antikörper gegen PAI-2
   14 Tage nach der Fusion wurden die Zellkulturüberstände der fusionierten Zellen mittels Enzymimmunoassay auf Antikörper gegen PAI-2 getestet: Polystyrol-Mikrotestplatten wurden mit 0.1 µg/ml PAI-2 in 0.1 mol/l NaCl, 0.1 mol/l Natriumacetat, pH 5.5, inkubiert (24 h, 4°C). Anschliessend wurden Zellkulturüberstände appliziert (2 h, 37°C), gefolgt von einer Inkubation mit einem peroxidase-konjugierten Antikörper gegen Maus-IgG. Als Substrat wurde eine Lösung von 0.1% (Gew/Vol) 2,2'-Azino-di-(3-ethyl-benzthiazolinsulfonat(6) und 0.12% (Vol/Vol) H₂O₂ in 0.1 mol/l Citronensäure, 0.1 mol/l Na₂HPO₄, pH 4.5, verwendet. Nach einer Inkubation von 30 min bei 37°C wurde die Absorption bei 405 nm gemessen. Zwischen den einzelnen Inkubationsschritten wurden die Vertiefungen der Testplatten mit PBS/^{R}Tween gewaschen.
d) Klonierung von antikörper-produzierenden Zellinien Zellen deren überstände im beschriebenen Enzymimmunoassay eine stark positive Reaktion (Absorption >1.5) zeigten, wurden nach der Methode der limitierenden Verdünnung kloniert. Dazu wurden ca. 60 Zellen in DMEM mit 20% FKS und 5% Human Endothelial Culture Supernatant (Fa. Costar) auf die 96 Kavitäten einer Zellkulturplatte verteilt. Einzelklone wurden mikroskopisch identifiziert und auf Antikörperproduktion getestet. Die Klonierung wurde zweimal wiederholt.
e) Reinigung von monoklonalen Antikörpern
   Klonale Zellinien wurden zur Produktion von Antikörpern in Roller-Flaschen überführt und in Iscove's Modified Dulbecco's Medium kultiviert. Zellen wurden durch Zentrifugation und Filtration über Papierfilter abgetrennt und mit Hilfe von Ultrafiltration etwa 10fach konzentriert. Das Konzentrat wurde über Protein A-^{R}Sepharose CL-4B gegeben und gebundenes IgG mit 0.2 mol/l Glycin/HCl, pH 3.0 eluiert. Die proteinhaltigen Fraktionen wurden gegen 0.1 mol/l Citrat, pH 6.5, dialysiert und mit Ultrafiltration auf ca. 5 mg/ml konzentriert.
f) Selektion von Antikörpern für die Immunaffinitätschromatographie
   Für die Immunaffinitätschromatographie geeignete monoklonale Antikörper wurden mit einem modifizierten Sandwich-ELISA selektiert. Verschiedene monoklonale Antikörper gegen PAI-2 (5 µg/ml in 0.1 mol/l NaCl, 0.1 mol/l Natriumacetat, pH 5.5) wurden jeweils auf drei Mikrotestplatten inkubiert (24 h, +4°C). Anschließend wurden alle so beschichteten Vertiefungen mit 1 µg/ml PAI-2 in PBS/^{R}Tween mit 2% Rinderserumalbumin inkubiert (2 h, 37°C) und eine Platte jeweils mit einer der folgenden Lösungen gewaschen (3 mal 5 min Inkubation): PBS, 3 mol/l NaSCN, destilliertes Wasser. Danach wurden die Platten mit einem peroxidase-konjugierten polyklonalen Antikörper gegen PAI-2 inkubiert (2h, 37°C). Die folgenden Schritte (Waschen, Substratinkubation, Messung) erfolgten wie unter Beispiel 1c beschrieben. Mit PBS gewaschene Platten dienten als Positivkontrolle für die Bindung von PAI-2 an die monoklonalen Antikörper, während mit NaSCN gewaschene Platten als Positivkontrolle für die Elution von PAI-2 von den von den monoklonalen Antikörpern dienten. Monoklonale Antikörper, die in der PBS-Kontrolle PAI-2 gebunden hatten (A₄₀₅ > 1.5) und nach dem Waschen mit destilliertem Wasser keine Reaktion zeigten (A₄₀₅ < 0.5), wurden für die Herstellung von Affinitätsgelen verwendet.
g) Herstellung von Affinitätsgelen
   Monoklonale Antikörper, nach Beispiel 1e gereinigt und nach 1f selektiert, wurden auf folgende Weise an Bromcyan-aktivierte ^{R}Sepharose 4B gekoppelt: 1.5 g Bromcyan-aktivierte ^{R}Sepharose 4B wurden in 1 mmol/l HCl suspendiert, in eine Chromatographie-Säule gefüllt und mit 300 ml HCl (1 mmol/l) gewaschen. Anschließend wurde die Säule mit 10 ml Kopplungspuffer (0.1 mol/l tri-Natriumcitrat, pH 6.5) äquilibriert und das Gel in ein verschließbares Gefäß mit 4ml einer Antikörperlösung (5 mg/ml in Kopplungspuffer) überführt und zwei Stunden bei 23°C geschüttelt. Danach wurde das Gel erneut in die Säule gegeben, mit 50 ml Kopplungspuffer gewaschen, in ein verschließbares Gefäß mit 20 ml Ethanolamin-HCl (1 mol/l, pH 8.0) gegeben und zwei Stunden bei 23°C geschüttelt. Das Gel wurde wieder in die Säule überführt und nacheinander mit je 100 ml 0.1 mol/l Natriumacetat, 1 mol/1 NaCl, pH 4.0 und 0.1 M Tris, 1 M NaCl, pH 8.0 gespült. Der Vorgang wurde 5 mal wiederholt. Zuletzt wurde das Gel mit PBS äquilibriert und war gebrauchsfertig.

### Beispiel 2

### Reinigung von PAI-2 aus einer Monocyten-Zellinie mittels Immunaffinitätschromatographie

Die Zellinie U-937 (ATCC CRL 1593) wurde in DMEM mit 10% FKS in Zellkulturflaschen (650cm²) kultiviert. Bei Erreichen einer Zelldichte von 1-2x10⁶/ml wurden die Zellen zweimal mit serumfreiem DMEM gewaschen und im gleichen Medium wieder auf Zellkulturflaschen ausgesät. Es wurden 30 ng/ml Phorbol-12-myristoyl-13-acetyl zugesetzt und die Zellen weitere 72 h inkubiert. 2 1 Zellüberstand wurden durch Klärfiltration gewonnen und durch Ultrafiltration etwa 40fach ankonzentriert. Das resultierende Konzentrat wurde über ein Affinitätsgel mit einem monoklonalen Antikörper (nach Beispiel 1g) gepumpt. Anschließend wurde nicht gebundenes Protein mit 100 ml Waschpuffer (1.0 mol/l NaCl, 0.02 mol/l Na₂HPO₄, pH 7.4) von der Säule gespült. PAI-2 wurde danach mit destilliertem Wasser eluiert. Das Eluat wurde gegen 0.15 mol/l NaCl, 0.02 mol/l Glycin, pH 7.2, dialysiert und über ConA-^{R}Sepharose gepumpt. Nach dem Waschen der ConA-^{R}Sepharose mit 0.15 mol/l NaCl, 0.02 mol/l Glycin, pH 7.2, wurde mit 0.5 mol/l Methyl-α-D-mannopyranosid in demselben Puffer eluiert. Durchlauf und Eluat der ConA-^{R}Sepharose wurden in der SDS-Polyacrylamid-Gelelektrophorese unter reduzierenden Bedingungen aufgetrennt. Die nicht gebundene Fraktion erschien als eine Bande mit 45.000 Dalton und entsprach der nicht glykosylierten Form von PAI-2. Die spezifische Aktivität betrug 170.000 E/mg. Das Eluat erschien ebenfalls als Einzelbande mit einem Molekulargewicht von 65.000 Dalton und entsprach dem glykosylierten PAI-2. Die spezifische Aktivität betrug 110.000 E/mg. Eine Einheit PAI-2 ist definiert als diejenige Menge, welche eine internationale Einheit Urokinase inhibiert.

### Beispiel 3

### Reinigung von PAI-2 aus rekombinanten Hefezellen mittels Immunaffinitätschromatographie

Die PAI-2 spezifische cDNA, wie sie von Antalis et al. (Proc.Natl.Acad.Sci.USA 85,985-989 (1988)) beschrieben ist, wurde in den Hefe/E.coli Shuttle-Vektor pEMBLyex4 (Cesareni and Murry, in: Genetic Engineering, Vol. 9,135-154, J.K. Setlow, ed., Plenum Press, 1987) dergestalt einkloniert, daß die Expression des PAI-2 unter der Kontrolle des regulierbaren GAL/CYC-Hybridpromotors steht. Das neue rekombinante Plasmid pPAI-2-A-10 wurde in Saccharomyces cerevisiae, Stamm CL3ABYS86, transformiert und transformierte Hefezellen auf Selektivmedium selektioniert. Die angewendeten molekularbiologischen und mikrobiologischen Techniken sind von Bröker et al. (Appl.Microbiol.Biotechnol. 34,756-764 (1991)) beschrieben. S. cerevisiae CL3ABYS86 [pPAI-2-A-10]-Transformanten wurden in Schüttelkulturen angezogen und die Zellen nach viertägiger Inkubation in einer Glaskugelmühle aufgeschlossen. Nach Zentrifugation des Lysates und Sterilfiltration konnte im Überstand in einem Urokinase-Hemmtest PAI-2 in einer Konzentration von ca. 45 mg/l, bezogen auf Kulturbrühe, nachgewiesen werden. 50 ml eines so gewonnenen Lysates wurden über ein Affinitätsgel mit einem monoklonalen Antikörper (nach Beispiel 1g) gepumpt. Anschließend wurde nicht gebundenes Protein mit 100 ml Waschpuffer (1.0 mol/l NaCl, 0.02 mol/l Na²HPO⁴, pH 7.4) von der Säule gespült. PAI-2 wurde danach mit destilliertem Wasser eluiert. Die spezifische Aktivität betrug 140.000-160.000 E/mg; das Protein erschien in der SDS-Polyacrylamid-Gelelektrophorese unter reduzierenden Bedingungen als eine Bande mit einem Molekulargewicht von 45.000 Dalton.

## Patentansprüche

1. Verfahren zur Gewinnung von reinem Plasminogen-Aktivator-Inhibitor 2 (PAI-2) in biologisch aktiver Form mittels Immunaffinitätschromatographie, dadurch gekennzeichnet, daß eine Lösung, die PAI-2 enthält mit einem an ein Trägermaterial gebundenen monoklonalen Antikörper (Affinitätsmaterial), gegen PAI-2 in Kontakt gebracht, Affinitätsmaterial und Flüssigkeit voneinander getrennt und PAI-2 vom Affinitätsmaterial in biologisch aktiver Form eluiert wird.

2. Verfahren nach Anspruch 1, dadurch gekennzeichnet, daß ein immunreaktives Fragment oder Derivat eines monoklonalen Antikörpers an das Trägermaterial gebunden ist.

3. Verfahren nach Anspruch 1, dadurch gekennzeichnet, daß mit einem Puffer mit einer Leitfähigkeit von ≦3 mS bei einem pH von 5.5 - 8.5 oder mit destilliertem Wasser eluiert wird.
